(19) Europäisches Patentamt
European Patent Office
Office européen des brevets

(11) **EP 3 498 855 B1**

## (12) EUROPEAN PATENT SPECIFICATION

(45) Date of publication and mention
of the grant of the patent:
**09.09.2020 Bulletin 2020/37**

(51) Int Cl.:
***C12P 19/04*** *(2006.01)*     ***C12N 1/12*** *(2006.01)*

(21) Application number: **18211136.9**

(22) Date of filing: **07.12.2018**

(54) **PROCESS FOR THE CULTIVATION OF MICROALGAE FOR THE PRODUCTION OF STARCH**

VERFAHREN ZUR KULTIVIERUNG VON MIKROALGEN ZUR HERSTELLUNG VON STÄRKE

PROCÉDÉ DE CULTURE DE MICROALGUES POUR LA PRODUCTION D'AMIDON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2017 IT 201700143274**

(43) Date of publication of application:
**19.06.2019 Bulletin 2019/25**

(73) Proprietor: **BIO-P S.r.l.**
**00156 Rome (IT)**

(72) Inventors:
• **Pagnanelli, Francesca**
**00055 Ladispoli (RM) (IT)**
• **Di Caprio, Fabrizio**
**00049 Velletri (RM) (IT)**

(74) Representative: **Sarpi, Maurizio et al**
**Studio Ferrario S.r.l.**
**Via Collina, 36**
**00187 Roma (IT)**

(56) References cited:
**WO-A1-2017/130106**     **WO-A2-2014/074769**

• **FABRIZIO DI CAPRIO ET AL: "Integrated biomass production and biodegradation of olive mill wastewater by cultivation of Scenedesmus sp.", ALGAL RESEARCH, vol. 9, 1 May 2015 (2015-05-01), pages 306-311, XP055293174, NL ISSN: 2211-9264, DOI: 10.1016/j.algal.2015.04.007**
• **Fabrizio Di Caprio ET AL: "Two Stage Process of Microalgae Cultivation for Starch and Carotenoid Production", CHEMICAL ENGINEERING TRANSACTIONS, 49, 1 April 2016 (2016-04-01), pages 415-420, XP055363932, DOI: 10.3303/CET1649070 Retrieved from the Internet: URL:http://www.aidic.it/cet/16/49/070.pdf [retrieved on 2017-04-11]**
• **DE LA HOZ SIEGLER H ET AL: "The dynamics of heterotrophic algal cultures", BIORESOURCE TECHNOLOGY, ELSEVIER, AMSTERDAM, NL, vol. 102, no. 10, 4 January 2011 (2011-01-04), pages 5764-5774, XP028407832, ISSN: 0960-8524, DOI: 10.1016/J.BIORTECH.2011.01.081 [retrieved on 2011-02-05]**

EP 3 498 855 B1

## Description

Field of application

The invention relates to the field of biosynthetic production of chemical compounds and biomolecules extracted from microalgae biomass.

The recognized biodiversity of microalgae provides a major source of biosynthesis pathways for the production of bioactive compounds, such as polysaccharides, proteins, fatty acids, carotenoids, pigments, antioxidants, polymer enzymes, peptides, sterols and biodiesel. In particular, the invention relates to the starch production field.

Prior art

Microalgae are aquatic unicellular plant microscopic organisms, which live singly or in colonies in fresh or salt water, whose growth, suitably favored by nutritive salts, light and carbon dioxide, can be considerably faster than that of terrestrial plants. This makes microalgae particularly suitable for the absorption of atmospheric $CO_2$, for the production of biofuels, for the purification of civil and agro-zootechnical waste and for the production of biomolecules.

Microalgae of various species are already produced industrially and used for the production of food supplements, feeds, pigments, omega 3 fatty acids, biomass for aquaculture and for the treatment of wastewater. The cultivation takes place in basins, tanks, photobioreactors and fermenters with different techniques and volumes according to the cultivated species and the particular applications.

The cultivation of microalgae for industrial purposes has a series of very interesting prerogatives, which are accompanied by various critical factors which still prevent an adequate exploitation of their potential, despite the efforts made in research and development.

Among the main advantages:

- very high growth rate;
- high proliferative capacity in nutrient rich waters, and therefore contribute to wastewater treatment processes;
- properties of absorbing $CO_2$ insufflated in the culture medium and transforming it into organic matter;
- possibility of growth even in hot climates and in salt water, without affecting the fresh water resources;
- possibility of being cultivated in limited surface areas without removing surfaces for agricultural cultivations for food or other economic activities;
- production of a homogeneous biomass, not divided into components with different characteristics, as happens for terrestrial plants with seeds, fruits, leaves, stems, roots).

The main issues are represented by:

- need for separation from the liquid phase;
- use of monospecies cultures in which contamination by other undesirable microalgae species or micro-organisms, insects and birds which eat them or prevent a proper development thereof must be avoided;
- cultivation scale, often quantitatively reduced, repeated at short time intervals instead of a more abundant one limited to one or two times a year.

Two main process configurations are currently used for the production of microalgae products: phototrophic cultivations and heterotrophic cultivations.

The phototrophic processes, although not conducted in axenic environment, are able to generally supply a biomass with a good degree of purity, which can also be used for nutraceutical applications. However, the need to ensure sufficient light supply requires the use of reactor configurations with a high surface/volume ratio (S/V), the use of transparent materials, an adequate supply of $CO_2$ and light. These factors lead to low biomass productivity compared to the installed equipment and to the process costs, with consequent high production costs. In addition, the need to work in an open environment, with reactors exposed to sunlight, greatly limits the application depending on the climatic characteristics and the location of installation of the system.

On the other hand, the problems of phototrophic plants can be summarized as follows:

a) low production per unit of land occupied,
b) low productivity and low microalgae concentrations achieved,
c) high production variability according to the climatic conditions, i.e. according to the period of the year and the geographic position of the plant,
d) use of poorly established reactor technologies,

e) high cost of the biomass produced.

The heterotrophic processes currently in use solve most of the problems of phototrophic implants (a-d), but current technologies require the use of strictly axenic cultivation conditions to allow the cultivation of microalgae in heterotrophy. To maintain these conditions, it is absolutely necessary to use sterilized equipment, along all the steps of the scale-up, from the laboratory to large-scale reactors, sterilizing in these steps every current entering the reactors (air, water, nutrients). If these strictly axenic conditions are not maintained, the algal biomass production process is strongly compromised both quantitatively and qualitatively. Maintaining these axenic conditions leads both to high production costs of biomass, even in heterotrophy, and to the generation of further problems.

The problems of current heterotrophic plants can be summarized as follows:

A. the selection of axenic microalgae cultures is necessary (more difficult, requires more expensive equipment and longer times).
B. use of equipment which is able to ensure the maintenance of the axenic conditions from the initial steps to the final ones of the scale-up (greater complexity, cost and susceptibility to malfunctions) .
C. high control (sterilization) required for each current entering the reactors.
D. applicability only to microalgae species able to live under axenic conditions (different microalgae species are not able to survive in the absence of some bacterial species).
E. high sensitivity to external perturbations (a single contamination event in one step of cultivation can shortly affect the whole process).
F. use only with sterilizable nutrients (hardly applicable to wastewater).
G. use only with microalgae species able to grow in heterotrophy.

From the foregoing it is apparent that the control of the growth of contaminants is currently the main limit to the heterotrophic cultivation of microalgae.

As described in the patent application US 2012/315678, some strains of microalgae, such as those of the genus Desmodesmus, accumulate up to 30%, or more, of starch in their organisms, with respect to their dry weight, when grown under suitable conditions. According to this invention, starch extracted from algae is produced by glucose hydrolysis.

The patent application US 2009/075353 describes a method for the production of ethanol, which comprises the extraction of starch obtained from unicellular green algae strains grown continuously, the algae produce and release extra-cellular starch in the culture medium, which is recovered and undergoes a process of saccharification and fermentation for the production of ethanol, while the algae are left in the culture medium to ensure the continuous process. This method of production is available and implementable anywhere in the world, from tropical areas to high latitude areas, it is not subjected to the control of natural climatic conditions but is carried out in an environment which can be monitored and controlled by humans.

The international patent application WO 2017/130106 describes a process for the production of starch from microalgae through cultivation in a phototrophic phase followed by a heterotrophic phase. The starch production takes place in the heterotrophic reactor, in which phenols of natural origin are added for the control of microbial contaminants and allow the heterotrophic growth of the microalgae. Microalgae specially selected for growth in the presence of phenols are used for this process. The nitrogen source is added at the beginning of the cultivation process. The carbon source is added in fed-batch during the cultivation process.

The process also involves the purification of the starch by a preliminary extraction of the lipids on the biomass obtained following the step of cultivation and separation from the culture medium. This biomass is first deprived of the lipids by solvent extraction, generating a residual biomass containing mainly starch and proteins. Then, it is subjected to a process of starch purification by hydrolysis of proteins in a basic environment. However, although there are data in the literature reporting the use of basic conditions for protein hydrolysis, our experimental data have shown that treatment in an alkaline environment, at different NaOH concentrations and at different temperatures, is not able to guarantee high efficiency of starch purification.

Therefore, the need for protocols of new heterotrophic processes for the production of biocomposites, in particular starch, is strongly felt, in which it is not necessary to work under axenic conditions (i.e. under sterile operating conditions which maintain a total absence of different microorganisms from the strain of cultivated algae), without requiring the use of phenols which can cause antimicrobial effects also on microalgae, thus reducing the yield of the process itself, with optimized conditions for the purification of starch.

The main drawbacks still present in the prior art processes mentioned above are overcome by the process of the invention described hereinafter, which constitutes an important advancement with respect to other processes based on the cultivation of microalgae used in the reference technological field, as it can be carried out under heterotrophic conditions and in the absence of strict axenia, using strains of microalgae, not necessarily tolerant to phenols, thus achieving higher productions of the order of 200 · $10^6$ cells/ml with respect to the processes of the prior art, from which starch is extracted with an optimized process based on the use of microwaves.

Summary of the invention

Therefore, the invention first relates to a method for the cultivation of microalgae under heterotrophic and non-axenic conditions, from the biomass of which starch is extracted. Said method allows obtaining an increased production of particularly starch-rich biomass from which, through an optimized extraction process, starch with high yield is obtained.

Secondly, the invention relates to a method for the cultivation of microalgae under heterotrophic and non-axenic conditions, from the biomass of which starch is obtained through various standardized extraction methods.

Thirdly, the invention relates to the particular method of extracting starch from a microalgae biomass.

Further objects of the invention will become apparent from the detailed description provided below.

Brief description of the drawings

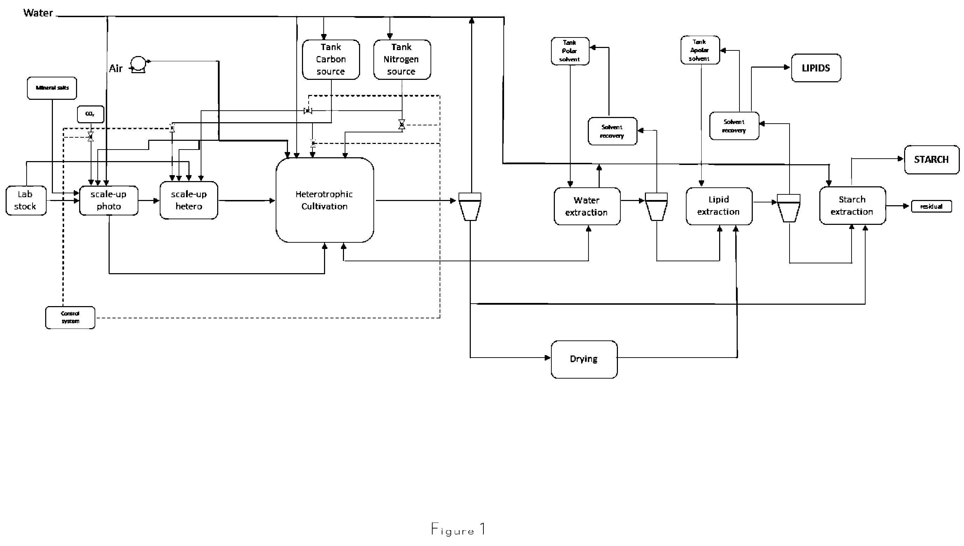

Figure 1 shows the flow chart of the microalgae cultivation process for the production of starch according to the invention.

Figure 2 shows the graph of the time trend of the cell concentration and of the biomass concentration in the sample grown in the reactor with fed-batch feeding methods with alternating additions of $NaNO_3$ and glucose.

Figure 3 shows the graph of the time trend of the cell concentration and of the biomass concentration in the sample grown in the reactor with fed-batch feeding methods with simultaneous additions of $NaNO_3$ and glucose.

Figure 4 shows the graph of the time trend of glucose concentration and nitrate concentration in the growth medium with fed-batch feeding methods with simultaneous additions of $NaNO_3$ and glucose.

Figure 5 shows the graph of the time trend of glucose concentration and nitrate concentration in the growth medium with fed-batch feeding methods with alternating additions of $NaNO_3$ and glucose.

Figure 6 shows the graph of the comparison of the concentration of carbohydrates accumulated by the biomass in the reactor during the fed-batch cultivation with the "simultaneous" and "alternating" feeding methods.

Figure 7 shows the concentration of bacterial contaminants expressed by colony-forming units (CFUs) inside the reactor during cultivation with the "simultaneous" and "alternating" nutrient feeding methods.

Figure 8 shows the graph representing the breaking efficiency of biomass for different times and powers of microwave treatment. The efficiency is calculated as the ratio between the mass present in the pellet obtained after centrifugation and the initial mass in the suspension.

Figure 9 shows the efficiency of carbohydrate extraction from the biomass of the two different algal strains investigated after 60 minutes of microwave irradiation at two different powers.

Detailed description of the invention

It is a first object of the invention to provide a method of cultivation of microalgae under heterotrophic and non-axenic conditions, preferably, but not exclusively, directed to the production of starch produced by microalgal metabolism.

In the present description, by the term microalgae it is meant micro-organisms, prokaryotes and eukaryotes, unicellular or organized in colonies and filaments, able to live using an autotrophic or photosynthetic metabolism, i.e. they feed through the process of photosynthesis, producing sugars and energy for their requirements from water, carbon dioxide and solar energy, specifically using light as a source of energy, $CO_2$ as a carbonaceous substrate, $H_2O$ as an electron donor.

Some microalgal species use a metabolism which allows them to use an organic substrate in the presence (mixotrophy) or in the absence (heterotrophy) of light, similar to bacteria and fungi. The particularity of the method according to the invention is that the microalgae used are grown under heterotrophic conditions.

The heterotrophic microalgal cultivation takes place in a reactor which is not exposed to light in which organic substrates are provided as energy and carbon source for the metabolism of microorganisms.

The method according to the invention also has the particularity of not requiring the development under axenic conditions, i.e. it is not required that monospecific cultivations are used for the production of biomass, and the possible contamination by other plant and animal micro-organisms does not represent a limitation to the development of the culture itself and does not require the adoption of chemical-physical measures to reduce the risk of contamination, which usually increase production costs to a significant extent.

Specifically, the process for the extraction of starch from a microalgae biomass obtained from cultivation under heterotrophic and non-axenic conditions according to the present invention may be divided into two main steps:

- a first step of heterotrophic cultivation of microalgae, whose purpose is the production of algal particularly starch-rich biomass, and
- a second step of separation and treatment of the obtained biomass, whose purpose is the extraction of the starch.

In turn, the cultivation step requires a preliminary start-up step, called scale-up, necessary to achieve the inoculum volumes required for the operation of the plant reactors.

A heterotrophic process for the cultivation of microalgae in the operative step requires the use of reactors having volume in the order of hundreds or thousands of cubic meters. This entails the need for an adequate preliminary cultivation start-up step which takes place in the plant, to allow micro-organisms to achieve the quantities of biomass required for reactor operation. The maintenance of the microalgae strain(s) can be carried out following the classic microbiological procedures used for the maintenance of the algal strain or species, i.e. essentially cultivations on solid media (with agar).

For the implementation of the method according to the invention, any microalgal strain capable of growing under heterotrophic conditions can be used. The species belonging to the Chlorophyceae class are preferred, and in particular species belonging to the genera Chlorella, Scenedesmus and Chlamydomonas. Any medium known to those skilled in the art and suitable for allowing the growth of the microalgae used can be used as a culture medium for the maintenance steps of the strain/species. Maintenance can be carried out by phototrophic, heterotrophic or mixotrophic, axenic and non-axenic cultivations. However, since unlike conventional processes, the process described herein does not require axenic inoculation, therefore non-axenic phototrophic cultivation is recommended in the maintenance step of the microalgae strain(s) because of its greater simplicity and because of the its widest spectrum of applicability (even to microalgal strains for which axenic cultivation is difficult or impossible).

*Preliminary scale-up step of the microalgae cultivation process for biomass production*

The scale-up step of the microalgae cultivation process for the production of biomass according to the present invention can be carried out both by phototrophic cultivation and heterotrophic cultivation.

In both cases, the operating method followed is the same, i.e. the microalgae suspension produced in small reactors is used as an inoculum for cultivation conducted in larger reactors, thus gradually increasing the volume of the cultivation up to achieve the volume necessary for carrying out the operating steps of the process, through a series of microalgal suspension cultivations, in a conventional type reactor, in succession, in which a microalgal suspension produced in a reactor is used as an inoculum for the subsequent cultivation conducted in the next reactor with a volume from 5 to 50 times that used to produce the respective inoculum, until achieving a final culture volume necessary for carrying out the heterotrophic cultivation step of microalgae.

The concentration of the microalgal inoculum used according to the invention for each step of the scale-up ranges from 0.01 to 1 g/L, preferably the concentration of the microalgal inoculum is 0.3 g/L. Each cultivation step is carried out until the concentration is increased, from initial to final, for a minimum value ranging between 5-50 times.

In a preferred embodiment of the invention, when the microalgal biomass concentration reaches values from 1 to 3 g/L, the microalgal suspension produced is transferred to the next larger volume reactor. The optimal volume difference between one reactor and the next one of larger volume is about 5-50 times. Once the microalgal suspension has been added to the larger volume reactor, fresh growth medium is added, and the suspension is started at the next cultivation step following the same procedure. For transferring microalgae suspensions from one reactor to another, any equipment known to those skilled in the art is used, such as volumetric pumps for transferring liquids.

In this preliminary scale-up step, the reactors are supplied with air at flow rates from 0.01 to 1 L/L/min, with an optimum value of 0.1 L/L/min.

In a particularly preferred embodiment of the invention, the pH value of the culture medium is maintained at a value from 4 to 12, preferably at the value of 8; this value is maintained by a feedback system by feeding with $CO_2$ in response to changes in the pH value with respect to the optimal value.

For the control of the temperature which according to the invention must be kept between 0 and 40 °C, preferably between 10 and 35 °C, even more preferable the temperature is $27 \pm 3$ °C, any system known to those skilled in the art may be used.

For the preparation of the growth media, it is recommended to use distilled water for small volumes used in the laboratory and of mains water for larger volumes, both for phototrophic and heterotrophic cultivations.

During each step of the scale-up, constant monitoring by means of a microscope and/or other techniques known to those skilled in the art to verify the purity of microalgae cultures must be carried out.

*a. Preliminary scale-up step of the microalgae cultivation process for biomass production with phototrophic method*

In the case in which the preliminary scale-up step of the microalgae cultivation process is carried out under a condition of phototrophy, the reactors used may have any conventional reactor configuration known to those skilled in the art, such as horizontal or vertical tubular photobioreactors, column photobioreactors, flat panels or open tanks. Phototrophy can be achieved either by solar or artificial lighting. Artificial lighting is recommended for the first steps of cultivation in the laboratory, i.e. when the culture volumes range from a few milliliters to a few liters, while solar lighting is preferably used for larger volumes, in outdoor or under greenhouse cultivation.

Any medium known to those skilled in the art and suitable for growing the cultivated microalgal species in question may be used as a growth medium, i.e. any medium capable of supplying the necessary nutrients to the metabolism of the microalgae in question, such as N, P, S, C, Mg, Fe, K, Ca and other micronutrients known to those skilled in the art in bioavailable forms.

Each phototrophic cultivation step during the scale-up is carried out for a time ranging from 3 to 30 days, preferably from 7 to 10 days. The phototrophic scale-up step can be prolonged until achieving the inoculum volume necessary for the start of the operating step of heterotrophic cultivation, or up to the inoculum in the heterotrophic reactors used for a second and more advanced step of the scale-up. In general, the use of phototrophic reactors is preferred up to volumes not exceeding a few hundred liters, leaving the designer the decision on the use or not of heterotrophic scale-up reactors, depending on the volume to be used in the operating step of the process.

In the last phototrophic cultivation step, i.e. before the transfer of biomass into the heterotrophic reactor where the production of starch-rich biomass occurs, it is necessary to control the concentration of the nitrogen source in the culture medium. This control is carried out by measuring at least once a day the concentration of the nitrogen source in the culture medium through the conventional methods known to those skilled in the art. An additional source of nitrogen is added to the medium if the detection shows that it does not reach the optimal value required.

The initial nitrogen concentration required in the medium must ensure a bioavailable nitrogen value above 0.01 g/L, preferably the bioavailable nitrogen must be between 0.04 and 0.25 g/L, even more preferably the initial concentration of nitrogen required in the culture medium during this cultivation step must ensure 0.065 g/L of bioavailable nitrogen.

As a nitrogen source, any bioavailable source for algae can be used, for example nitrate or ammonium salts, amino acids, urea or a mixture thereof. However, in a particularly preferred embodiment of the invention, the nitrogen source consists of nitrate salts (e.g. $NaNO_3$ and $KNO_3$). This cultivation step is interrupted when the concentration of nitrogen in the medium drops below a value of about 1-5 mg/L, after which the microalgal suspension is transferred to the heterotrophic reactor.

The transfer of the algal biomass thus obtained in the reactor in which the subsequent heterotrophic cultivation of microalgae takes place for the production of starch-rich algal biomass is carried out no more than 12-24 hours from the achievement of the minimum concentration of nitrogen in the medium. Any means known to those skilled in the art, such as volumetric pumps, may be used to transfer the algal suspension produced in the heterotrophic reactor.

In another embodiment of the invention, the biomass obtained in the preliminary scale-up step of the process is transferred into the heterotrophic reactor even if the concentration of the nitrogen source is higher than 5 mg/L, in this case it is necessary to perform alternative operations, for example it is necessary to separate the solid biomass from the culture medium. The separation of the solid phase from the liquid phase can be carried out by any means known by those skilled in the art, for example it can be obtained by centrifugation, provided that it can provide a separation efficiency of the order of 90-100%. The solid biomass thus separated can be directly introduced into the heterotrophic reactor, while the residual supernatant, consisting of the exhausted growth medium, can be recirculated and reused in the upstream phototrophic cultivation steps (after integration of the consumed nutrients), or sent to wastewater treatment.

### b. Preliminary scale-up step of the microalgae cultivation process for biomass production with heterotrophic method

In a further embodiment of the invention, the preliminary scale-up step of the microalgae cultivation process for the production of biomass is carried out under heterotrophic conditions.

In the case in which the scale-up step of the cultivation is carried out in heterotrophic reactors, the procedure is carried out according to the conditions and methods of the operative heterotrophic cultivation described below. In the scale-up step, the heterotrophic configuration is recommended if operating process volumes larger than a few $m^3$ are to be achieved. Scale-up under heterotrophic conditions can be performed by using one or more heterotrophic reactors of different volume, based on the final volume designed for the operating step of the plant.

According to the invention, the heterotrophic step, whether it be the operating step in which the starch-rich biomass is produced or the scale-up step, can be started by adding the nitrogen source to the medium used for the cultivation from the beginning; in this case, any preliminary phototrophic cultivation should be continued for at least 3-5 days after achieving concentrations lower than 5 mg/L of nitrogen in the growth medium. This measure is necessary to allow the accumulation of organic carbon reserves to the microalgae, which are essential for the subsequent growth in the heterotrophic reactor in the step characterized by the presence of the nitrogen source and lack of the organic carbon source.

As a dilution ratio for heterotrophic cultivation, a dilution ratio similar to that used for the phototrope scale-up steps is preferred, i.e. the volume of the heterotrophic reactor must be about 5-50 times the volume of the phototrophic/heterotrophic reactor used to produce the inoculum. Unlike the phototropic scale-up, the part conducted with heterotrophic reactors can be carried out up to the achievement of biomass concentrations in the reactor ranging from 2-200 g/L, preferably 10-20 g/L. Once this concentration is achieved, the microalgal suspension produced can be transferred into a heterotrophic reactor with a volume of 5-50 times higher.

***Heterotrophic cultivation step of microalgae for the production of starch-rich biomass***

The inoculum obtained as described in the preliminary start-up or scale-up step is transferred to the heterotrophic reactor where the operating step of the cultivation aimed at the production of starch-rich biomass is carried out, which takes place through a fed-batch process carried out under non-axenic conditions, in which the microalgal metabolism is favored by the addition of a substrate source of organic carbon and a source of nitrogen in the reactor. Surprisingly, it has been observed that the addition to the system in reaction of the organic carbon source substrate and the nitrogen source alternately allows controlling the development of fungal and bacterial contaminations.

In the present description, fed-batch culture means a semi-open system, that is, open only at the input, but not at the output, therefore also called variable volume. Generally, the semi-open fed-batch system begins as a closed system, in which all the nutrients necessary for cell growth, known as culture medium as a whole, are provided at the beginning of cultivation, then, once an adequate amount of biomass has been achieved, the fed-batch process begins in semi-open mode in which a growth-limiting substrate is continuously added to the culture, which determines the increase of biomass in proportion to the amount of nutrients supplied, until other factors take over causing a slowing down of growth, such as the lack of oxygen; at this point, it is convenient to slow down the flow of nutrients until the system is stopped. This expedient allows extending the growth time of microorganisms before reaching the steady state. The fed-batch culture prevents problems of inhibition by toxic cellular metabolism products and the accumulation of mutations, as well as decreasing the possibility of contamination.

According to the invention, the inoculum used has a concentration ranging from 0.1 to 100 g/L, preferably from 1 to 50 g/L, even more preferably the inoculum used has a concentration of 10 g/L which, as described above, in a preferred embodiment, derives from a scale-up cultivation conducted in the presence of nitrogen-rich medium, but may also derive from a scale-up cultivation conducted in the absence of nitrogen. In the case of inoculum deriving from a cultivation step in the presence of nitrogen, the initial step of the fed-batch heterotroph process is a cultivation step with the addition of organic carbon. Otherwise, i.e. in case of inoculum deriving from a cultivation step in the absence of nitrogen, the initial cultivation step in the fed-batch heterotrophic process begins with the addition of the nitrogen source.

The heterotrophic cultivation for the production of starch-rich biomass according to the present invention is carried out in a conventional reactor operating under fed-batch conditions. The non-axenic condition under which the process according to the invention takes place means that it is not necessary that the reactor and its contents are subjected to sterilization, therefore it is not required that the reactor be made up of autoclavable material; preferably, the heterotrophic cultivation step takes place in a conventional stainless-steel reactor.

The reactor in which the heterotrophic step of the process takes place is provided with the following inlets for:

- introducing the organic substrate,
- introducing the nitrogen source,
- introducing air,
- introducing the inoculum,
- introducing the shaft for stirring,
- introducing the control probes (minimum two: pH and temperature),
- introducing the basic solution,
- introducing the acidic solution.

As a stirring means, any system capable of ensuring the stirring of the microalgae culture having concentrations ranging from 1-100 g/L can be used. It is not necessary to use systems able to ensure high hermeticity.

According to the method according to the invention, neither the use of autoclavable probes nor the use of openings for the entry of high-hermetic probes is required. At least two probes are required, one for the pH control and one for the temperature. If necessary, additional probes can be provided to control other useful parameters, such as for example the concentration of the organic substrate, the concentration of the nitrogen source, dissolved oxygen, cell density.

The injection of air into the reactor can be ensured by any means known to those skilled in the art, capable of ensuring the introduction of air with sufficient head and flow rate such as to ensure sufficient solubilization of the oxygen. The use of air filtration systems is not strictly necessary, although they are recommended.

The basic and acidic solutions used to control the pH value are stored in special tanks and delivered to the reactor by activating/deactivating a volumetric pump controlled by a feedback control system. For the control of the pH value in the cultivation medium, preferably HCl and NaOH are used at a concentration in the range of 1 to 5 M, but other solutions known to those skilled in the art may also be used. The use of acids or bases with nitrogen (e.g. ammonium salts or nitric acid) and organic acids or bases (e.g. acetic acid, acetate salts) is not recommended.

The plant in which the process according to the invention is carried out does not require lines for the introduction of high temperature steam, through which reactors and components are generally sterilized.

The reactor is provided with the following outputs:

- reactor emptying valve,
- air outlet,
- valve(s) for sampling.

In the plant, in addition to the reactor, there must be tanks for the storage of the growth medium.

For storage of the nitrogen source a conventional tank is used to store the solution comprising the nitrogen source. The tank must prevent the light from passing therethrough, therefore it must be made of a material, or provided with an external covering, suitable for this purpose. This tank is connected to the reactor by means of pipes configured in such a way as to prevent the passage of light and a conventional volumetric pump capable of ensuring a controllable flow.

For storage of the organic carbon substrate a conventional tank is used to store the solution comprising the organic carbon source. The tank is connected to the reactor and the solution delivered by means of a conventional volumetric pump able to ensure a controllable flow.

As previously described, the initial inoculum derives from a scale-up cultivation which can be carried out in the presence of nitrogen-rich medium, or in the absence of nitrogen. In the case of inoculum deriving from a cultivation step in the presence of nitrogen, the initial step of the fed-batch heterotroph process is a cultivation step with organic carbon. Otherwise, i.e. in case of inoculum deriving from a cultivation step in the absence of nitrogen, the initial cultivation step in the fed-batch heterotrophic process begins with the addition of the nitrogen source to the growth medium.

*Cultivation step with organic carbon*

A volume of solution taken from the organic carbon storage tank is added to the inoculum present in the reactor. The volume of solution with organic carbon added to the inoculum is less than or equal to the volume of the inoculum, preferably it has a volume lower than 1/10 of the volume of the inoculum. The solution stored in the organic carbon storage tank is produced by mixing the following products:

- Water: mains water is preferred, however distilled water, with a degree of variable purity, waste water or mixtures thereof may also be used. In the case of use of mains water and/or waste water and/or mixtures thereof, the concentration of nutrients supplied by them must comply with the values indicated below.
- Organic carbon source: as an organic carbon source, any source which can be used by cultivated microalgae may be used, such as sugars (glucose, lactose, galactose, fructose, sucrose, etc.), alcohols and polyalcohols (ethanol, glycerol) and organic acids (acetic acid, citric acid, propionic acid). A mixture of several molecules can also be used. A source containing a percentage of N greater than 0.1% (calculated as N/C) cannot be used. The organic carbon source can be provided by solubilizing the solid organic substrate, at different degrees of purity, or by adding waste water with a high C/N ratio, i.e. capable of maintaining the N/C ratio lower than 0.1% (with C referred to organic carbon alone). The concentration of organic carbon can range between 0.4 and 500 gC/L, preferably the concentration of organic carbon is 100 gC/L.
- Macronutrients: phosphorus, sulfur, potassium, calcium and magnesium can be added in the form of salts (e.g. phosphates, sulfates, chlorides, etc.) and/or organic molecules. In any case, bioavailable sources for microalgae must be used. Molecules containing N may also be used if the added amount does not lead to values of N in the storage tank higher than 0.1% with respect to the organic carbon in solution. The concentrations of macronutrients must be related to the concentration of the carbon source. For each g/L of organic carbon (gC/L), macronutrients must have the following concentration ranges: P = 0.001-0.07 g/L, S = 0.025-0.04 g / L, K = 0.02-0.03 g/L, Mg = 0.01-0.07 g/L, Ca = 0.025-0.04 g/L.
- Micronutrients: iron, copper, zinc, molybdenum, boron, manganese can be added in the form of salts (for example sulfates, chlorides, etc.) bioavailable for microalgae. For each g/L of organic carbon, macronutrients must have the following optimal concentrations: Fe = 1-2 mg/L, Cu = 0.06-0.07 $\mu$g/L, Mo = 0.6-0.7 $\mu$g/L, Zn = 6-7 $\mu$g/L, Mn = 5-6 $\mu$g/L, B = 5-6 $\mu$g/L. Sodium generally does not need to be added because it is already sufficiently present in the mains water in the wastewater and/or in the salts used to provide the other nutrients.

In the case of use of organic carbon sources available in solid form, these can also be added directly into the reactor, and the tank for storage of the organic carbon source replaced with a tank for the storage of the micronutrient and macronutrient solution (the latter will be configured as described above except for the lack of the organic carbon source).

The organic carbon source solution may also consist of water alone and an organic carbon source in solution. In this case, the other nutrients (macro and micro) can be added at the beginning of the fed-batch process, or at intervals during the fed-batch process as selected by the designer. In this case, additional storage tanks and inlets into the reactor will have to be provided.

The quantity of organic carbon added to the inoculum must be dosed according to the concentration of biomass of the inoculum using the following formula:

$$m_{OC} = \frac{m_{b,in}}{Y_{b,OC}}$$

Where $m_{OC}$ denotes the mass of organic carbon to be added to the heterotrophic reactor at the beginning of the cultivation step with organic carbon, $m_{b,in}$ denotes the biomass present in the heterotrophic reactor at the beginning of the cultivation step with organic carbon (inoculum mass), while $Y_{b,OC}$ denotes the yield in g of microalgal biomass produced for g of organic carbon used. The factor $Y_{b,OC}$ is specific for the microalgal strain and the type of organic carbon added.

Following the addition of the organic substrate, the cultivation is maintained for a time ranging from 1 to 30 days, preferably 5 days, under constant stirring, at controlled pH and temperature and continuous air supply. When carrying out the invention, those skilled in the art can easily select the stirring and air flow configurations capable of maintaining conditions of adequate oxygen exchange and an adequate homogenization of the suspension. The pH ranges from 4 to 12, a value between 6-7 is preferred.

The system operates at a temperature ranging from 0 to 40 °C, preferably at a temperature ranging from 10 to 35 °C, even more preferably the temperature at which the cultivation takes place upon addition of an organic substrate is 27 ± 3 °C. The end of the cultivation step with organic carbon coincides with the depletion of organic carbon in solution. This condition is necessary to prevent the proliferation of bacteria.

*Cultivation step with nitrogen*

At the end of the cultivation step with organic carbon, one proceeds with the addition of the nitrogen source. A volume of solution taken from the nitrogen source storage tank is added to the reactor. A value of less than 1/10 of the volume of the suspension in the reactor is used, preferably a volume of less than 1/100 of the volume of suspension in the reactor. The amount of nitrogen added in this step ranges from 5 to 100 mg for each gram of biomass present in the reactor, preferably 40 mg/g biomass. This step is maintained for a time ranging from 1 to 7 days, preferably for 2-3 days. All the other parameters of the reactor are kept unchanged with respect to the organic carbon cultivation step. The end of the nitrogen cultivation step coincides with the depletion of the nitrogen in solution. This condition is necessary to prevent the proliferation of bacteria. As a nitrogen source, any source of bioavailable inorganic nitrogen for the microalgae used may be used. However, the preferred configuration provides for the use of nitrate salts such as for example $NaNO_3$ and $KNO_3$.

The two cultivation steps, that with nitrogen and that with organic substrate, are alternated with one another. The strategy according to which the two alternate types of heterotrophic cultivation, that with the addition of the nitrogen source and that with the addition of organic carbon substrate, can be repeated n times until the predetermined concentration, and/or quantity, of biomass is achieved. The starch is produced during the cultivation step with organic carbon, so at the end of this step the biomass is collected.

In a particular embodiment of the invention, a new fed-batch cultivation cycle can be initiated by using as biomass the biomass deriving from the scale-up process both from a step with nitrogen and without nitrogen; in a particularly preferred alternative embodiment, a new fed-batch cultivation cycle can be started by using a part of the suspension produced at the end of the organic carbon cultivation step which is used as an inoculum for the new fed-batch cultivation. In this case, the biomass of the inoculum of the new fed-batch cultivation will have to start cultivation with a step of nitrogen addition, as it derives from a cultivation step with organic carbon.

To determine the amount of the nitrogen source and the amount of organic carbon source to be added at the beginning of each step, during the cultivation it is necessary to monitor the concentration of biomass in the reactor over time. For this purpose, any system known to those skilled in the art may be used. For example, an optical density detector may be used in the case of an online control. In the case of off-line control, on the other hand, sampling from the reactor is carried out at least at the beginning and at the end of each cultivation step, measurements of the biomass concentration are carried out by determining the dry weight (e.g. vacuum filtration).

During cultivation it is advisable, even if not strictly necessary, to monitor the concentration of nitrogen and carbon in solution. The values to be achieved are the complete depletion of the nitrogen source at the end of the nitrogen step, i.e. before the carbon source is introduced, and the complete depletion of the carbon source at the end of the carbon step, i.e. before the nitrogen source. These conditions should be achieved within the indicated times, but it is recommended to monitor the concentrations of nitrogen and organic carbon for a greater optimization of the process times.

At the end of the heterotrophic cultivation step, a microalgae biomass is then obtained, particularly enriched in starch, which will be extracted by following successive steps of the process according to a first aspect of the invention. However, as those skilled in the art can easily understand, the process described above can be used to obtain microalgae biomass from which it is possible to extract not only starch, but also other types of biological macromolecules of industrial interest using already standardized extraction processes known in the prior art. This process for obtaining a biomass through the heterotrophic and non-axenic cultivation of microalgae constitutes a second aspect of the invention.

*Solid-liquid biomass separation step and lipid extraction*

The microalgal suspension obtained in the heterotrophic cultivation step of the described process deriving from the fed-batch cultivation unit is subsequently sent to a solid-liquid separation unit. The separation of the solid phase from the liquid phase can be carried out by any means known to those skilled in the art, for example it can be obtained by centrifugation. The solid phase of the concentrated biomass having a humidity point from 70 and 90% is fed into an extraction reactor with solvent. This reactor is of the conventional type, suitable for carrying out extraction processes with solvent or solvent mixture, selected according to the nature of the extract to be obtained. For the first extraction provided by the process according to the invention, the microalgae biomass is fed into the reactor and suspended in a polar organic solvent completely miscible in water. For this purpose, short chain (C<6) alcohols or polyalcohols, such as methanol, ethanol, glycerol, propanol or isopropanol, or mixtures thereof, may be used; in particular, ethanol is the preferred extraction solvent. For the extraction, the solid liquid ratio is $100 \pm 20$ L of solvent for each kg of biomass (intended as dry weight). The suspension is left under stirring for a period of 5-10 minutes at a temperature ranging from 25 to 35 °C, preferably at a temperature of 30 °C.

After this extraction step, the suspension obtained is sent back to a liquid solid separation unit, where the separation can be carried out by any means deemed suitable by those skilled in the art, generally centrifugation is preferred for the separation. The obtained liquid phase is sent to a solvent recovery unit, which can be recycled for a subsequent extraction cycle, while the remaining water phase can be sent to the wastewater treatment, to the subsequent starch extraction step or recycled in the microalgae cultivation steps. The biomass obtained after this first solvent extraction step is fed into another solvent extraction reactor, inside which the extraction solvent or the mixture of lipid extraction solvents is introduced. For this second extraction cycle, the use of a mixture of a polar solvent and an apolar one is preferred, in particular hexane-2-propanol is preferred. Alternatively, other mixtures known to those skilled in the art for their applicability to the lipid extraction process (e.g. hexane-methanol, dichloromethane-methanol, hexane-ethanol, dichloromethane-ethanol, etc.) may also be used. The optimal ratio for this extraction step is $500 \pm 100$ L of solvent per kg of dry weight biomass. The optimal temperature is $30 \pm 5$ °C. This extraction process is carried out for a time ranging from 10 minutes to 6 hours, preferably for 1 hour. At the end of this second extraction step, the suspension is again sent to a solid-liquid separation unit, in which separation of the water-ethanol solution takes place. The residual biomass after the extraction of the lipid fraction is sent to the starch extraction reactor, while the organic lipid-solvent mixture is sent to a unit for lipid-solvent separation, at a temperature from 25 to 75 °C, preferably at a temperature of 30 °C. The recovered solvents can be reused for a subsequent extraction step. The operating temperatures, both for the two solvent extraction steps, and for the separation step of the solvent(s) from the lipids, must be selected based on the final use of the lipid fraction. In the case in which it is intended to produce a microalgae oil which is rich in carotenoids, the optimal temperature for the two extraction steps is a low temperature, preferably around 30 °C. However, in the case of different uses, which do not necessarily require low temperatures to safeguard the integrity of the final product, i.e. carotenoids, higher temperatures may be used, up to 75 °C, preferably 60-75 °C. In an alternative option, lipid extraction may also be carried out on dried biomass by removing the water with conventional thermal methods known by those skilled in the art (e.g. heating in ovens, freeze drying, spray drying, etc.).

*Starch extraction step*

The residual biomass obtained after the extraction of the lipids is sent to the reactor for the extraction of the starch. According to the present invention, the extraction of starch from biomass obtained from the process described above and subjected to treatment for the extraction of the lipids is carried out in a microwave-irradiated reactor. For this purpose, any reactor known by those skilled in the art and suitable for the purpose and capable of generating an optimum frequency of 2.45 GHz, and a power in the range of 5-50 kW/L may be used, preferably the power is equal to 33 kW/L.

Inside this reactor, together with the biomass, water is added, for an optimal quantity of 10 L per kg of biomass, however, larger quantities of water can also be used. Extraction times over 5 minutes, preferably 60 minutes. During this step, the microwaves break the residual biomass, separating the starch from the other cellular components (proteins, lipid residues and wall polysaccharides) and bringing it in aqueous solution in the form of thermoplastic starch. In this step, the system reaches temperatures of 80-100 °C. At the end of the process, the suspension is separated with a further separation treatment of the solid-liquid phases, at the end of which the starch in the form of thermoplastic starch is contained in the liquid fraction, while the waste material is sent to valorization treatments (e.g. feed or biogas production).

The starch extraction operation can also be carried out on the biomass as such, i.e. on the biomass obtained from the liquid solid separation carried out after the heterotrophic cultivation, without having undergone a lipid fraction extraction process.

In summary, the present invention provides for:

- a first object related to a process for producing starch from a biomass obtained through the cultivation of microalgae under heterotrophic and non-axenic conditions, comprising the following steps:

   a) preliminary start-up step of the heterotrophic cultivation process of microalgae (so-called scale-up) ;
   a1)
   possible further separation step from the biomass culture medium obtained in the preliminary start-up step a) with a separation efficiency of 90-100%;
   b) transfer into the heterotrophic reactor;
   c) heterotrophic fed-batch cultivation step of microalgae wherein a substrate supplying a source of organic carbon and a substrate supplying a source of nitrogen is alternately fed to the reaction system;
   d) separation of the solid phase of the microalgal biomass obtained in step c);
   e) first extraction of the microalgae biomass from the solid phase obtained in step d) with a watersoluble polar organic solvent;
   f) separation of the solid phase obtained in step e), recovery and recycling of solvent and water;
   g) second extraction of the microalgal biomass from the solid phase obtained in step f) with a mixture of polar solvent and apolar solvent.
   h) liquid-solid separation and recovery of the solid phase obtained in step g),
   i) extraction of the starch from the biomass obtained in h) in a reactor irradiated by microwaves;
   j) liquid-solid separation and recovery of the liquid phase containing the starch;

- a second object related to a process for the cultivation of microalgae under heterotrophic and non-axenic conditions in order to obtain a microalgae biomass suitable for subsequent extraction/isolation/purification processes of starch which process comprises following steps:

   a) preliminary start-up step of the heterotrophic cultivation process of microalgae (so-called scale-up);
   a1) possible further separation step from the biomass culture medium obtained in the preliminary start-up step a) with a separation efficiency of 90-100%;
   b) transfer into the heterotrophic reactor;
   c) heterotrophic fed-batch cultivation step of microalgae wherein a substrate supplying a source of organic carbon and a substrate supplying a source of nitrogen is alternately fed to the reaction system;
   d) separation of the solid phase of the microalgal biomass obtained in step c);

- a third object related to a process for the extraction of starch from a microalgae biomass, however obtained, comprising the following steps:

   a) extraction conducted in a reactor irradiated with microwaves;
   b) liquid-solid separation and recovery of the liquid phase containing the starch.

Experimental part

The invention will now be described hereinafter by way of non-limiting illustration with particular reference to some examples carried out on a laboratory scale, but indicative of the advantages obtained with the process according to the present invention.

**Example 1** - **Heterotrophic cultivation**

The experiment described below was carried out using biomass inoculations from a pilot plant. The latter were maintained for three days under stirring in the laboratory under conditions of phototrophy in the presence of 1 g/L of sodium nitrate. The amount of biomass needed to achieve the desired concentration was centrifuged to remove the supernatant and washed in order to eliminate the remaining sodium nitrate. The flasks were then brought to a volume of 500 mL using a medium with the following composition: $K_2HPO_4$ (300 mg/L), $MgSO_4$ x $6H_2O$ (306 mg/L), $CaCl_2$ x $2H_2O$ (36 mg/L), citric acid (6 mg/L), ammonium citrate (60 mg/L), EDTA (1 mg/L), $NaCO_3$ (20 mg/L), $H_3BO_3$ (0.061 mg/L), $MnSO_4$ x $H_2O$ (0.17 mg/L), $ZnSO_4$ x $7H_2O$ (0.287 mg/L), $(NH_4)_6Mo_7O_{24}$ x $4H_2O$ (0.0125 mg/L), $CuSO_4$ x $5H_2O$ (0.0025 mg/L). Glucose and $NaNO_3$ were added to the medium, preparing two different starting configurations:

- *Scenedesmus obliquus* 1 g/L, glucose 10 g/L, sodium nitrate ($NaNO_3$) 1 g/L;
- *Scenedesmus obliquus* 1 g/L, glucose 2 g/L, sodium nitrate ($NaNO_3$) 0 g/L.

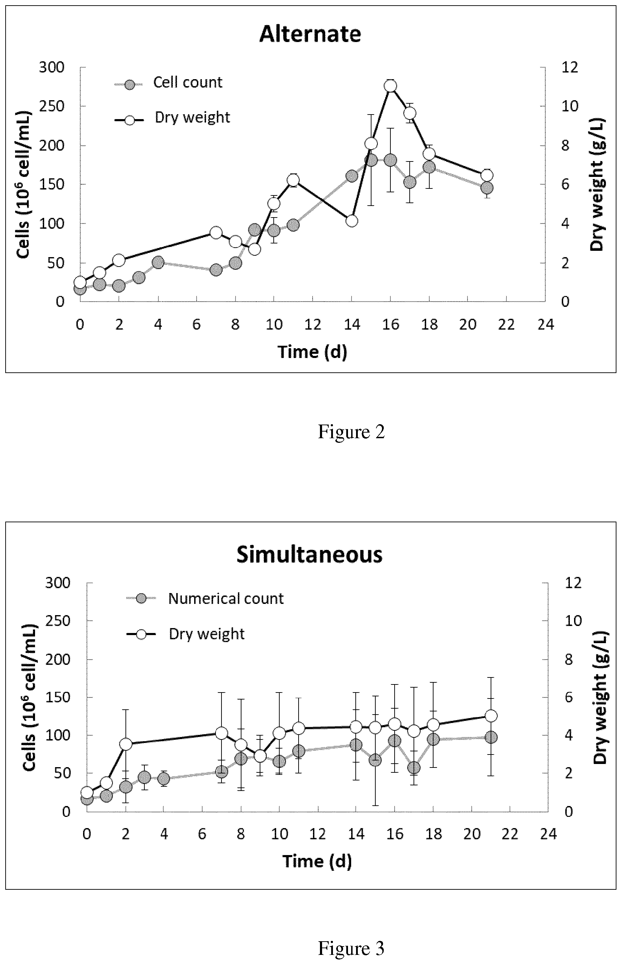

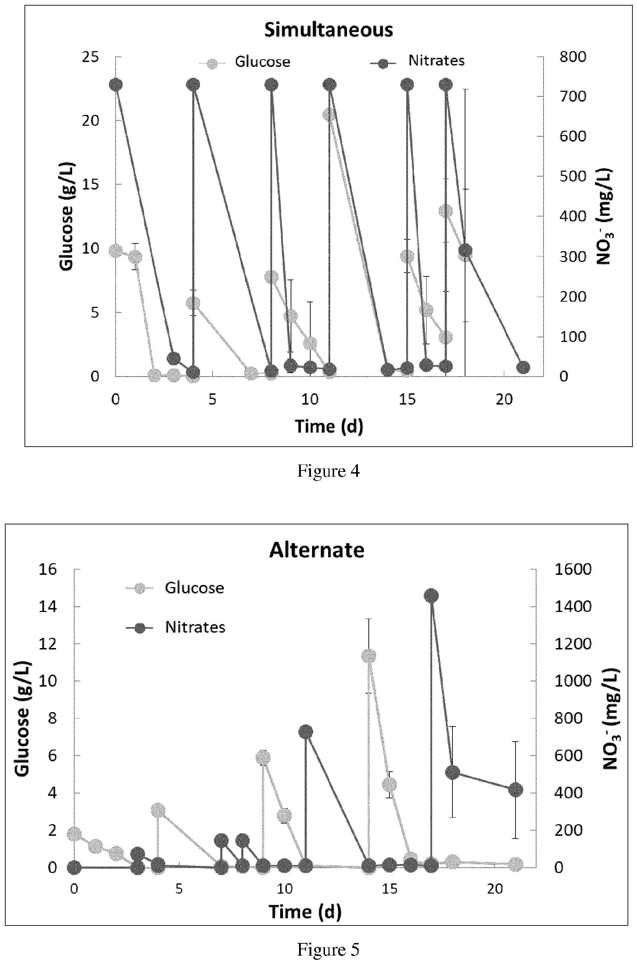

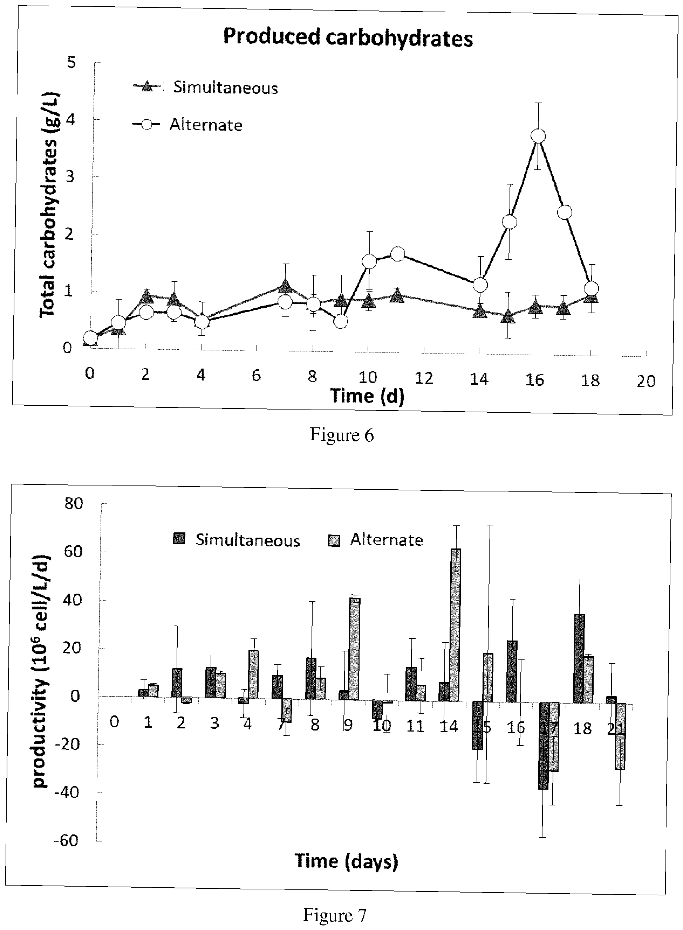

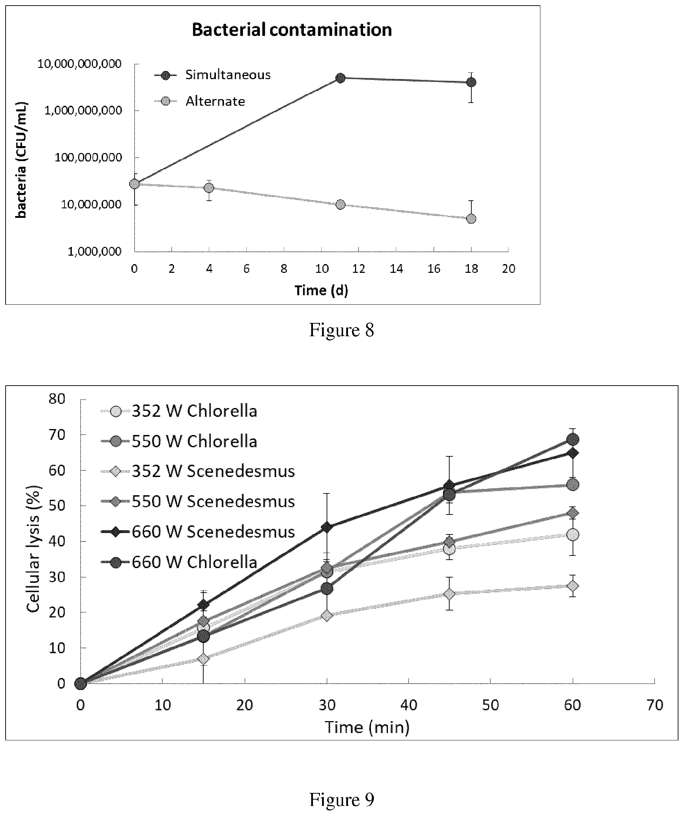

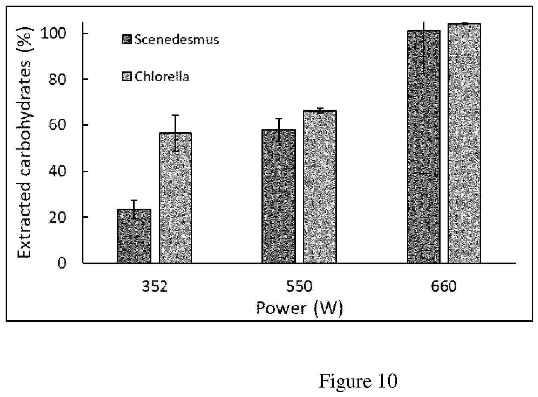

The experiments were carried out in biological replicate taking different inocula from the phototrophic pilot plant.
The pH value of the growth medium, without sodium nitrate, was first brought to 7 before its use. The pH was checked daily, always bringing it to 6.5 through the addition of HCl or NaOH. The usual growth monitoring operations were carried out, such as cell count and dry weight analysis, were then carried out. Furthermore, the concentration of glucose and nitrates ($NO_3^-$) in solution was periodically monitored. Continuous monitoring of glucose and nitrate concentration is necessary to understand when to add the substrates again. In the so-called "simultaneous" samples, once the quantity of organic and nitrogenous substrate initially supplied was finished, glucose and sodium nitrate were added again simultaneously, with concentrations equal to the initial ones (10 g/L of glucose and 1 g/L of $NaNO_3$). This process was carried out until the end of the experiment. In the so-called "alternate" samples, glucose and sodium nitrate were alternately added with weighted concentrations from time to time depending on the concentration achieved by the biomass, considering a specific theoretical growth yield equal to 0.3 g (biomass)/g (glucose). Also in this case, the additions were made as soon as the solution component was finished (for example, as soon as the glucose was finished, sodium nitrate was added; then, as soon as the latter was finished, glucose was added, etc.).
In order to evaluate bacterial contamination, growth tests on plate were performed, with selective medium for the growth of bacteria (LB) and fungi (YPD).
Through a qualitative and quantitative evaluation through a count of the colony-forming units (CFU) it was possible to evaluate the trend of microbial contamination during the experimentation in the different samples.
The results obtained in the experiment are shown in the graphs in figures 2 to 10 and summarized in table 1, which presents the comparison between different parameters for the assessment of the production efficiency of cells and biomass with respect to the consumption of glucose and nitrates, for the two different tested feeding strategies.

Table 1

|  | Alternate | Simultaneous | Advantage compared to simultaneous |
|---|---|---|---|
| $C_{max}$, cell (cell· $10^6$/mL) | 180 ± 40 | 90 ± 40 | +100% |
| $C_{max}$,biom (g/L) | 11.1 ± 0.3 | 5 ± 2 | +120% |
| Pmax (g/L/day) | 4.0 ± 0.1 | 2 ± 2 | +100% |
| $Y_{cell/gluc}$ (cell· $10^6$/g gluc) | 6 ± 2 | 1.2 ± 0.7 | +400% |
| $Y_{biom/gluc}$ (g biom/g gluc) | 0.38 ± 0.01 | 0.07 ± 0.04 | +440% |
| $Y_{cell/NO3}$-(cell·$10^6$/g $NO_3^-$) | 90 ± 20 | 17 ± 9 | +430% |
| $Y_{biom/NO3}$-(g biom/g $NO_3^-$) | 10.0 ± 0.3 | 1.9 ± 0.5 | +900% |

$C_{max,cell}$ = maximum cell concentration achieved. $C_{max,biom}$ = maximum concentration of biomass achieved. $P_{max}$ = maximum biomass productivity achieved. $Y_{cell/gluc}$ = average yield of cells produced with respect to the glucose consumed. $Y_{biom/gluc}$ = average yield of biomass produced with respect to the glucose consumed. $Y_{cell/NO3}$-= average yield of cells produced with respect to the nitrates consumed. $Y_{biom/gluc}$ = average yield of biomass produced with respect to the nitrates consumed.

**Example 2** - **Extraction of starch from microalgal biomass**

Twenty mL of algal suspension (3 g/L) were placed in 500 mL glass bottles. The bottles placed in the microwave without cap and the microwave started at the desired power (352, 550 or 660 W). After 5 minutes of treatment the suspension was centrifuged, the supernatant put aside and the biomass resuspended in 20 mL of cold water. The suspension was treated again in the microwave. After each resuspension of the biomass, the absorbance at 680 nm was determined at the spectrophotometer for the measurement of dry weight. The process was repeated up to reach 60 minutes of total treatment.

On the supernatants produced at each centrifugation step, the concentration of extracted starch was determined by measuring the total quantity of carbohydrates by means of saccharification and glucose analysis. The process was performed after protein precipitation with acetone. The experiment was conducted on both *Scenedesmus obliquus* and *Chlorella emersonii* biomass.

**Claims**

1. A process for the production of starch produced by microalgae through a heterotrophic cultivation process **characterized in that** it is carried out under non-axenic conditions comprising the following steps:

   a) preliminary start-up step of the heterotrophic cultivation process of microalgae (so-called. scale-up), carried out under either phototrophic or heterotrophic conditions, or both;
   a1) possible further separation step from the biomass culture medium obtained in the preliminary start-up step a) with a separation efficiency of 90-100%;
   b) transfer into a heterotrophic reactor;
   c) heterotrophic fed-batch cultivation step of microalgae wherein a substrate supplying a source of organic carbon and a substrate supplying a source of nitrogen is alternately fed to the reaction system;
   d) separation of the solid phase of the microalgal biomass obtained in step c);
   e) first extraction of the microalgae biomass from the solid phase obtained in step d) with a watersoluble polar organic solvent;
   f) separation of the solid phase obtained in step e), recovery and recycling of solvent and water;
   g) second extraction of the microalgal biomass from the solid phase obtained in step f) with a mixture of polar solvent and apolar solvent.
   h) liquid-solid separation and recovery of the solid phase obtained in step g),
   i) extraction of the starch from the biomass obtained in step h) in a reactor irradiated by microwaves; and
   j) liquid-solid separation and recovery of the liquid phase containing the starch.

2. The process according to claim 1, wherein the used microalgae belong to any microalgal strain able of growing under heterotrophic conditions, preferably of species belonging to the taxonomic class of Chlorophyceae, to the genera Chlorella, Scenedesmus and Chlamydomonas.

3. The process according to claims 1 and 2, wherein the preliminary start-up step a) is carried out under phototrophic or heterotrophic conditions or both, using a series of conventional reactors in succession, wherein a microalgal suspension produced in a reactor is used as inoculum for the subsequent cultivation conducted in the next reactor having a volume 5 to 50 times greater than the volume of the reactor used to produce the respective inoculum in the previous scale-up step, up to reaching a final culture volume sufficient for carrying out the heterotrophic cultivation of microalgae step c).

4. The process according to claim 3, wherein the preliminary start-up step a) is carried out under phototrophic conditions and is conducted up to reaching in the culture medium the minimum concentration of nitrogen below 5 mg/L, is stopped when the concentration of nitrogen in the culture medium is higher than 5 mg/L, and within 24 hours the obtained microalgal biomass is transferred to the reactor wherein the subsequent heterotrophic cultivation takes place.

5. The process according to claim 3, wherein the solid phase of the biomass obtained in the preliminary start-up step is further separated from the liquid phase with separation efficiency between 90 and 100%, preferably by centrifugation.

6. The process according to claim 3, wherein the preliminary start-up step a) is carried out under heterotrophic fed-batch conditions in a series of reactors in succession whose inoculum has a biomass concentration ranging from 0.1 to 100 g/L, preferably between 1 and 50 g/L.

7. The process according to claim 6 wherein in the preliminary start-up step a) carried out under heterotrophic conditions, the inoculum derives from a nitrogen cultivation step and the fed-batch heterotrophic process starts with a cultivation step with added organic carbon in the medium, or the inoculum derives from an organic carbon cultivation step and the initial step of the fed-batch heterotrophy process starts with a cultivation step with nitrogen added in the medium.

8. The process according to claim 7, wherein the nitrogen substrate added into the reactor derives from an aqueous solution stored in a nitrogen storage reservoir containing as nitrogen source an inorganic nitrogen salt in a form bioavailable for the microalgae, ammonium salts, nitrate salts, preferably solutions of $NaNO_3$ and $KNO_3$.

9. The process according to claim 7, wherein the organic carbon substrate added to the reactor derives from a solution stored in an organic carbon storage reservoir which comprises: water, sugars, alcohols, polyalcohols, organic acids alone or variously combined among them, macronutrients and micronutrients bioavailable for microalgae.

10. The process according to claim 9 wherein:

    - water is mains water, distilled water, waste water or mixtures thereof,
    - macronutrients are selected from the group of: phosphorus, sulfur, potassium, calcium, magnesium phosphates, sulfates, chlorides and organic molecules,
    - micronutrients are selected from the group of: iron, copper, zinc, molybdenum, boron, manganese and their sulfate and chloride salts.

11. The process according to claim 10, wherein for each organic carbon g/L the macronutrients have the following concentration ranges:

    P = 0.001-0.07 g/L,
    S = 0.025-0.04 g/L,
    K = 0.02-0.03 g/L,
    Mg = 0.01-0.07 g/L,
    Ca = 0.025-0.04 g/L,

    and the micronutrients have the following concentration ranges:

    Fe = 1-2 mg/L,
    Cu = 0.06-0.07 $\mu$g/L,
    Mo = 0.6-0.7 $\mu$g/L,
    Zn = 6-7 $\mu$g/L,
    Mn = 5-6 $\mu$g/L,
    B = 5-6 $\mu$g/L.

12. The process according to claims 10-11 wherein all macro- and micro-nutrients of the solution stored in the organic carbon storage reservoir are added to the reactor at the beginning of the fed-batch process.

13. The process according to any one of claims 10-12 wherein the amount of carbon added to the inoculum of the heterotrophic cultivation step is dosed as a function of the inoculum biomass concentration according to the following relation:

$$m_{OC} = \frac{m_{b,in}}{Y_{b,OC}}$$

wherein:

    $m_{OC}$ indicates the organic carbon mass to be added to the heterotrophic reactor at the beginning of the organic carbon cultivation step,
    $m_{b,in}$ indicates the biomass present in the heterotrophic reactor at the beginning of the cultivation step with organic carbon (inoculum mass),
    $Y_{b,OC}$ indicates the yield in g of microalgal biomass produced for g of organic carbon used, it is specific for the microalgal strain and the type of organic carbon added.

**14.** The process according to any one of claims from 10 to 12 wherein, following the addition of the organic carbon substrate, the cultivation is maintained for a time ranging from 1 to 30 days, under constant stirring, at a pH between 4 and 12 and temperature between 0 and 40 ° C, with continuous air supply, until the organic carbon in solution is exhausted.

**15.** The process according to any one of claims 10 to 14, wherein at the end of the cultivation step with organic carbon, the nitrogen cultivation step starts by adding a volume of solution from the nitrogen storage reservoir, wherein the amount of added nitrogen ranges between 5 and 100 mg for each gram of biomass present in the reactor, where the cultivation is maintained for a time between 1 and 7 days, under constant stirring, at a pH between 4 and 12 and the temperature between 0 and 40 °C, with continuous air supply, until the nitrogen in solution is exhausted.

**16.** The process according to claim 7 wherein in the fed-batch mode the nitrogen substrate and the organic carbon substrate are directly added in solid form into the reactor.

**17.** The process according to claim 1, wherein the heterotrophic fed-batch cultivation step of microalgae in c) is carried out as described in claims 7 to 16 with reference to the heterotrophic cultivation in the start-up step a).

**18.** The process according to claim 17 wherein in the heterotrophic cultivation step in c) the organic carbon cultivation step and the nitrogen cultivation step alternate up to reaching of the predetermined biomass concentration and/or amount, and said heterotrophic cultivation step ends with cultivation with organic carbon.

**19.** The process according to claim 1, wherein the extraction of step e) is carried out from a microalgae biomass obtained in step c) having a humidity point between 70 and 90% in a reactor by extraction in the presence of a completely miscible in water polar organic solvent, wherein the suspension is left under stirring for 5-10 minutes at a temperature ranging from 25 to 35 ° C, said completely miscible in water polar organic solvent being a short chain alcohol or polyalcohol (C <6), preferably methanol, ethanol, glycerol, propanol, isopropanol or mixtures thereof, and wherein solvent and biomass are respectively in a ratio of $100 \pm 20$ L of solvent per kg of dry weight biomass.

**20.** The process according to claim 1, wherein the extraction of step g) is carried out in an extraction reactor in the presence of a mixture of a polar solvent and an apolar solvent, wherein the biomass suspension is left for an hour at a temperature between 25 and 35 °C.

**21.** The process according to claim 20 wherein the mixture of polar solvent and apolar solvent is selected from the group of: hexane-2-propanol, hexane-methanol, dichloromethane-methanol, hexane-ethanol, dichloromethane-ethanol, and wherein solvent and biomass are respectively in a ratio of $500 \pm 100$ L of solvent per kg of dry weight biomass.

**22.** The process according to claim 1, wherein the starch extraction of step i) takes place in an irradiated microwave reactor capable of generating an optimum frequency of 2.45 GHz, and a power ranging from 5 to 50 kW / L, preferably equal to 33 kW/L, wherein water is added, at least 10 L for each kg of dry weight biomass, and is conducted for a time exceeding 5 minutes, preferably for 60 minutes.


**Patentansprüche**

**1.** Verfahren zur Herstellung von Stärke, die durch Mikroalgen durch ein heterotrophes Kultivierungsverfahren erzeugt wird, **dadurch gekennzeichnet, dass** es unter nicht-axenischen Bedingungen durchgeführt wird, umfassend die folgenden Schritte:

a) Vorbereitender Anfangsschritt des heterotrophen Kultivierungsverfahrens von Mikroalgen (sogenanntes scale-up), das entweder unter phototrophen oder heterotrophen Bedingungen oder unter beiden durchgeführt wird;
a1) Möglicher weiterer Trennungsschritt von dem in dem vorbereitenden Anfangsschritt a) erhaltenen Biomasse-Nährboden mit einer Trenneffizienz von 90-100%;
b) Überführung in einen heterotrophen Reaktor;
c) heterotropher Fed-Batch-Kultivierungsschritt von Mikroalgen, wobei ein Substrat, das eine Quelle für organischen Kohlenstoff liefert, und ein Substrat, das eine Quelle für Stickstoff liefert, abwechselnd in das Reaktionssystem eingespeist werden;
d) Abtrennung der festen Phase der in Schritt c) erhaltenen Mikroalgen-Biomasse;
e) erste Extraktion der Mikroalgen-Biomasse aus der in Schritt d) erhaltenen festen Phase mit einem wasserlöslichen polaren organischen Lösungsmittel;

f) Abtrennung der in Schritt e) erhaltenen festen Phase, Rückgewinnung und Rückführung von Lösungsmittel und Wasser;

g) zweite Extraktion der Mikroalgen-Biomasse aus der in Schritt f) erhaltenen festen Phase mit einem Gemisch aus polarem Lösungsmittel und apolarem Lösungsmittel;

h) Flüssig-Fest-Trennung und Rückgewinnung der in Schritt g) erhaltenen festen Phase;

i) Extraktion der Stärke aus der in Schritt h) erhaltenen Biomasse in einem Reaktor, der mit Mikrowellen bestrahlt wird; und

j) Flüssig-Fest-Trennung und Rückgewinnung der flüssigen Phase, die die Stärke enthält.

2. Verfahren nach Anspruch 1, wobei die verwendeten Mikroalgen zu jedem Mikroalgenstamm gehören, der unter heterotrophen Bedingungen wachsen kann, vorzugsweise von Arten, die zur taxonomischen Klasse der Chlorophyceae gehören, zu den Gattungen Chlorella, Scenedesmus oder Chlamydomonas.

3. Verfahren nach den Ansprüchen 1 und 2, wobei der vorbereitende Anfangsschritt a) unter phototrophen oder heterotrophen Bedingungen oder beiden unter Verwendung einer Reihe von konventionellen Reaktoren nacheinander durchgeführt wird, wobei eine in einem Reaktor hergestellte Mikroalgensuspension als Inokulum für die nachfolgende Kultivierung verwendet wird, die in dem nächsten Reaktor durchgeführt wird, der ein Volumen aufweist, das 5- bis 50-mal größer ist als das Volumen des Reaktors, der zur Herstellung des jeweiligen Inokulums im vorhergehenden scale-up-Schritt verwendet wurde, bis zum Erreichen eines Endkulturvolumens, das ausreicht, um die heterotrophe Kultivierung der Mikroalgen im Schritt c) durchzuführen.

4. Verfahren nach Anspruch 3, wobei der vorbereitende Anfangsschritt a) unter phototrophen Bedingungen durchgeführt wird und bis zum Erreichen der minimalen Stickstoffkonzentration im Kulturmedium unter 5 mg/L durchgeführt wird, gestoppt wird, wenn die Stickstoffkonzentration im Kulturmedium höher als 5 mg/L ist, und innerhalb von 24 Stunden die erhaltene Mikroalgen-Biomasse in den Reaktor überführt wird, in dem die anschließende heterotrophe Kultivierung stattfindet.

5. Verfahren nach Anspruch 3, wobei die feste Phase der in dem vorbereitenden Anfangsschritt erhaltenen Biomasse weiter von der flüssigen Phase mit einem Trennwirkungsgrad zwischen 90 und 100%, vorzugsweise durch Zentrifugation, getrennt wird.

6. Verfahren nach Anspruch 3, wobei der vorbereitende Anfangsschritt a) unter heterotrophen Fed-Batch-Bedingungen in einer Reihe von aufeinanderfolgenden Reaktoren durchgeführt wird, deren Inokulum eine Biomassekonzentration im Bereich von 0,1 bis 100 g/L, vorzugsweise zwischen 1 und 50 g/L, aufweist.

7. Verfahren nach Anspruch 6, wobei in dem vorbereitenden Anfangsschritt a), der unter heterotrophen Bedingungen durchgeführt wird, das Inokulum aus einem Stickstoff-Kultivierungsschritt stammt und das heterotrophe Fed-Batch-Verfahren mit einem Kultivierungsschritt mit zugesetztem organischem Kohlenstoff im Medium beginnt, oder das Inokulum aus einem organischen Kohlenstoff-Kultivierungsschritt stammt und der Anfangsschritt des Fed-Batch-Heterotrophie-Verfahrens mit einem Kultivierungsschritt mit zugesetztem Stickstoff im Medium beginnt.

8. Verfahren nach Anspruch 7, wobei das in den Reaktor zugegebene Stickstoffsubstrat aus einer wässrigen Lösung stammt, die in einem Stickstoffvorratsbehälter gelagert ist, welcher als Stickstoffquelle und anorganisches Stickstoffsalz in einer für die Mikroalgen bioverfügbaren Form, Ammoniumsalze, Nitratsalze, vorzugsweise Lösungen von $NaNO_3$ und $KNO_3$, enthält.

9. Verfahren nach Anspruch 7, wobei das organische Kohlenstoffsubstrat, das dem Reaktor zugegeben wird, von einer Lösung stammt, die in einem organischen Kohlenstoffspeicher gespeichert ist, welcher umfasst: Wasser, Zucker, Alkohole, Polyalkohole, organische Säuren allein oder unterschiedlich unter ihnen kombiniert, Makronährstoffe und Mikronährstoffe, die für die Mikroalgen bioverfügbar sind.

10. Verfahren nach Anspruch 9, wobei:

- Wasser Leitungswasser, destilliertes Wasser, Abwasser oder Mischungen davon ist,
- Makronährstoffe ausgewählt werden aus der Gruppe von: Phosphor, Schwefel, Kalium, Kalzium, Magnesiumphosphate, Sulfate, Chloride und organische Moleküle,
- Mikronährstoffe ausgewählt werden aus der Gruppe von: Eisen, Kupfer, Zink, Molybdän, Bor, Mangan und deren Sulfat- und Chloridsalze.

**11.** Verfahren nach Anspruch 10, wobei für jeden organischen Kohlenstoff g/L die Makronährstoffe die folgenden Konzentrationsbereiche aufweisen:

P = 0,001-0,07 g/L,
S = 0,025-0,04 g/L,
K = 0,02-0,03 g/L,
Mg = 0,01-0,07 g/L,
Ca = 0,025-0,04 g/L,
und die Mikronährstoffe die folgenden Konzentrationsbereiche aufweisen:

Fe = 1-2 mg/L,
Cu = 0,06-0,07 $\mu$g/L,
Mo = 0,6-0,7 $\mu$g/L,
Zn = 6-7 $\mu$g/L,
Mn = 5-6 $\mu$g/L,
B = 5-6 $\mu$g/L.

**12.** Verfahren nach den Ansprüchen 10-11, wobei alle Makro- und Mikronährstoffe der in dem organischen Kohlenstoffspeicher gespeicherten Lösung zu Beginn des Fed-Batch-Verfahrens in den Reaktor gegeben werden.

**13.** Verfahren nach einem der Ansprüche 10-12, wobei die Menge an Kohlenstoff, die dem Inokulum des heterotrophen Kultivierungsschritts zugegeben wird, in Abhängigkeit von der Inokulum-Biomassekonzentration gemäß der folgenden Beziehung dosiert wird:

$$m_{OC} = \frac{m_{b,in}}{Y_{b,OC}}$$

wobei:

$m_{OC}$ die organische Kohlenstoffmasse angibt, die dem heterotrophen Reaktor zu Beginn des Kultivierungsschrittes des organischen Kohlenstoffs zuzusetzen ist,
$m_{b,in}$ die Biomasse angibt, die zu Beginn des Kultivierungsschrittes mit organischem Kohlenstoff (Inokulum-Masse) im heterotrophen Reaktor vorhanden ist,
$Y_{b,OC}$ die Ausbeute in g der Mikroalgen-Biomasse angibt, die für g des verwendeten organischen Kohlenstoffs erzeugt wird, sie ist spezifisch für den Mikroalgen-Stamm und die Art des zugesetzten organischen Kohlenstoffs.

**14.** Verfahren nach einem der Ansprüche 10 bis 12, wobei nach der Zugabe des organischen Kohlenstoffsubstrats die Kultivierung über einen Zeitraum von 1 bis 30 Tagen unter ständigem Rühren bei einem pH-Wert zwischen 4 und 12 und einer Temperatur zwischen 0 und 40 °C bei kontinuierlicher Luftzufuhr aufrechterhalten wird, bis der organische Kohlenstoff in Lösung erschöpft ist.

**15.** Verfahren nach einem der Ansprüche 10 bis 14, wobei am Ende des Kultivierungsschrittes mit organischem Kohlenstoff der Stickstoffkultivierungsschritt durch Zugabe eines Lösungsvolumens aus dem Stickstoffvorratsbehälter beginnt, wobei die Menge des zugegebenen Stickstoffs zwischen 5 und 100 mg für jedes Gramm der im Reaktor vorhandenen Biomasse liegt, wobei die Kultivierung über einen Zeitraum zwischen 1 und 7 Tagen unter ständigem Rühren bei einem pH-Wert zwischen 4 und 12 und einer Temperatur zwischen 0 und 40 °C bei kontinuierlicher Luftzufuhr aufrechterhalten wird, bis der Stickstoff in Lösung erschöpft ist.

**16.** Verfahren nach Anspruch 7, wobei im Fed-Batch-Modus das Stickstoffsubstrat und das organische Kohlenstoffsubstrat direkt in fester Form in den Reaktor gegeben werden.

**17.** Verfahren nach Anspruch 1, wobei der heterotrophe Fed-Batch-Kultivierungsschritt von Mikroalgen in c) wie in den Ansprüchen 7 bis 16 mit Bezug auf die heterotrophe Kultivierung in dem Anfangsschritt a) beschrieben durchgeführt wird.

**18.** Verfahren nach Anspruch 17, wobei in dem heterotrophen Kultivierungsschritt in c) der Kultivierungsschritt mit organischem Kohlenstoff und der Stickstoffkultivierungsschritt bis zum Erreichen der vorbestimmten Biomassekonzentration und/oder -menge abwechseln und der besagte heterotrophe Kultivierungsschritt mit der Kultivierung mit organischem Kohlenstoff endet.

**19.** Verfahren nach Anspruch 1, wobei die Extraktion von Schritt e) aus einer in Schritt c) erhaltenen Mikroalgen-Biomasse mit einem Feuchtigkeitspunkt zwischen 70 und 90% in einem Reaktor durch Extraktion in Gegenwart eines vollständig mit Wasser mischbaren polaren organischen Lösungsmittels durchgeführt wird, wobei die Suspension 5-10 Minuten lang bei einer Temperatur im Bereich von 25 bis 35 °C unter Rühren belassen wird, wobei das vollständig mit Wasser mischbare polare organische Lösungsmittel ein kurzkettiger Alkohol oder Polyalkohol (C <6) ist, vorzugsweise Methanol, Ethanol, Glycerin, Propanol, Isopropanol oder Mischungen davon, und wobei Lösungsmittel und Biomasse jeweils in einem Verhältnis von 100 $\pm$ 20 L Lösungsmittel pro kg Biomasse in Trockengewicht vorliegen.

**20.** Verfahren nach Anspruch 1, wobei die Extraktion von Schritt g) in einem Extraktionsreaktor in Gegenwart eines Gemisches aus einem polaren Lösungsmittel und einem apolaren Lösungsmittel durchgeführt wird, wobei die Biomassesuspension eine Stunde lang bei einer Temperatur zwischen 25 und 35 °C belassen wird.

**21.** Verfahren nach Anspruch 20, wobei das Gemisch aus polarem Lösungsmittel und apolarem Lösungsmittel ausgewählt ist aus der Gruppe von: Hexan-2-propanol, Hexan-Methanol, Dichlormethan-Methanol, Hexan-Ethanol, Dichlormethan-Ethanol, und wobei Lösungsmittel und Biomasse jeweils in einem Verhältnis von 500 $\pm$ 100 L Lösungsmittel pro kg Biomasse Trockengewicht vorliegen.

**22.** Verfahren nach Anspruch 1, wobei die Stärkeextraktion von Schritt i) in einem bestrahlten Mikrowellenreaktor stattfindet, der in der Lage ist, eine optimale Frequenz von 2,45 GHz und eine Leistung im Bereich von 5 bis 50 kW / L, vorzugsweise gleich 33 kW/L, zu erzeugen, wobei Wasser zugegeben wird, mindestens 10 L für jedes kg Biomasse in Trockengewicht, und für eine Zeitdauer von mehr als 5 Minuten, vorzugsweise für 60 Minuten, durchgeführt wird.

## Revendications

**1.** Procédé pour la production d'amidon produit par des microalgues par le biais d'un procédé de culture hétérotrophe **caractérisé en ce qu'**il est mis en œuvre dans des conditions non axéniques comprenant les étapes suivantes :

a) étape de démarrage préliminaire du procédé de culture hétérotrophe de microalgues (que l'on appelle accroissement), mise en œuvre dans des conditions soit phototrophes soit hétérotrophes, ou les deux ;
a1) possible étape de séparation supplémentaire du milieu de culture de biomasse obtenu lors de l'étape de démarrage préliminaire a) avec une efficacité de séparation de 90 à 100 % ;
b) transfert dans un réacteur hétérotrophe ;
c) étape de culture hétérotrophe à alimentation discontinue de microalgues, dans lequel un substrat approvisionnant en source de carbone organique et un substrat approvisionnant en source d'azote sont alimentés en alternance dans le système de réaction ;
d) séparation de la phase solide de la biomasse de microalgues obtenue lors de l'étape c) ;
e) première extraction de la biomasse de microalgues à partir de la phase solide obtenue lors de l'étape d) avec un solvant organique polaire soluble dans l'eau ;
f) séparation de la phase solide obtenue lors de l'étape e), une récupération et un recyclage de solvant et d'eau ;
g) seconde extraction de la biomasse de microalgues à partir de la phase solide obtenue lors de l'étape f) avec un mélange de solvant polaire et de solvant apolaire ;
h) séparation liquide-solide et une récupération de la phase solide obtenue lors de l'étape g),
i) extraction de l'amidon à partir de la biomasse obtenue lors de l'étape h) dans un réacteur irradié par des micro-ondes ; et
j) séparation liquide-solide et une récupération de la phase liquide contenant l'amidon.

**2.** Procédé selon la revendication 1, dans lequel les microalgues utilisées appartiennent à une quelconque souche de microalgues apte à croître dans des conditions hétérotrophes, de préférence d'une espèce appartenant à la classe taxonomique des Chlorophyceae, aux genres Chlorella, Scenedesmus et Chlamydomonas.

**3.** Procédé selon les revendications 1 et 2, dans lequel l'étape de démarrage préliminaire a) est mise en œuvre dans des conditions phototrophes ou hétérotrophes ou les deux, à l'aide d'une série de réacteurs conventionnels qui se suivent, dans lequel une suspension de microalgues produite dans un réacteur est utilisée en tant qu'inoculum pour la culture consécutive menée dans le réacteur suivant possédant un volume 5 à 50 fois plus grand que le volume du réacteur utilisé pour produire l'inoculum respectif lors de l'étape d'accroissement précédente, jusqu'à atteindre un volume de culture final suffisant pour mettre en œuvre la culture hétérotrophe de microalgues de l'étape c).

4. Procédé selon la revendication 3, dans lequel l'étape de démarrage préliminaire a) est mise en œuvre dans des conditions phototrophes et est menée jusqu'à atteindre dans le milieu de culture la concentration minimale d'azote inférieure à 5 mg/l, est arrêtée lorsque la concentration d'azote dans le milieu de culture est supérieure à 5 mg/l et en 24 heures la biomasse de microalgues obtenue est transférée au réacteur, dans lequel la culture hétérotrophe consécutive a lieu.

5. Procédé selon la revendication 3, dans lequel la phase solide de la biomasse obtenue lors de l'étape de démarrage préliminaire est en outre séparée de la phase liquide avec une efficacité de séparation entre 90 et 100 %, de préférence par centrifugation.

6. Procédé selon la revendication 3, dans lequel l'étape de démarrage préliminaire a) est mise en œuvre dans des conditions hétérotrophes à alimentation discontinue dans une série de réacteurs qui se suivent dont l'inoculum possède une concentration de biomasse allant de 0,1 à 100 g/l, de préférence entre 1 et 50 g/l.

7. Procédé selon la revendication 6, dans lequel lors de l'étape de démarrage préliminaire a) mise en œuvre dans des conditions hétérotrophes, l'inoculum est dérivé d'une étape de culture à l'azote et le procédé hétérotrophe à alimentation discontinue démarre avec une étape de culture avec du carbone organique ajouté dans le milieu, ou l'inoculum est dérivé d'une étape de culture au carbone organique et l'étape initiale du procédé d'hétérotrophie à alimentation discontinue démarre avec une étape de culture avec de l'azote ajouté dans le milieu.

8. Procédé selon la revendication 7, dans lequel le substrat d'azote ajouté dans le réacteur est dérivé d'une solution aqueuse stockée dans un réservoir de stockage d'azote contenant en tant que source d'azote un sel d'azote inorganique sous une forme biodisponible pour les microalgues, des sels d'ammonium, des sels de nitrate, de préférence des solutions de $NaNO_3$ et de $KNO_3$.

9. Procédé selon la revendication 7, dans lequel le substrat de carbone organique ajouté au réacteur est dérivé d'une solution stockée dans un réservoir de stockage de carbone organique qui comprend : de l'eau, des sucres, des alcools, des polyalcools, des acides organiques seuls ou combinés diversement parmi eux, des macronutriments et des micronutriments biodisponibles pour les microalgues.

10. Procédé selon la revendication 9, dans lequel :

   - l'eau est de l'eau du robinet, de l'eau distillée, des eaux usées ou des mélanges de celles-ci,
   - les macronutriments sont sélectionnés à partir du groupe constitué par : le phosphore, le soufre, le potassium, le calcium, les phosphates de magnésium, les sulfates, les chlorures et les molécules organiques,
   - les micronutriments sont sélectionnés à partir du groupe constitué par : le fer, le cuivre, le zinc, le molybdène, le bore, le manganèse et leurs sels de sulfate et de chlorure.

11. Procédé selon la revendication 10, dans lequel pour chaque g/l de carbone organique les macronutriments possèdent les plages de concentration suivantes :

   P = 0,001 à 0,07 g/l,
   S = 0,025 à 0,04 g/l,
   K = 0,02 à 0,03 g/l,
   Mg = 0,01 à 0,07 g/l,
   Ca = 0,025 à 0,04 g/l,
   et les micronutriments possèdent les plages de concentration suivantes :

   Fe = 1 à 2 mg/l,
   Cu = 0,06 à 0,07 $\mu$g/l,
   Mo = 0,6 à 0,7 $\mu$g/l,
   Zn = 6 à 7 $\mu$g/l,

Mn = 5 à 6 μg/l,
B = 5 à 6 μg/l.

**12.** Procédé selon les revendications 10 et 11, dans lequel tous les macronutriments et micronutriments de la solution stockée dans le réservoir de stockage de carbone organique sont ajoutés au réacteur au début du procédé à alimentation discontinue.

**13.** Procédé selon l'une quelconque des revendications 10 à 12, dans lequel la quantité de carbone ajoutée à l'inoculum de l'étape de culture hétérotrophe est dosée en fonction de la concentration de biomasse d'inoculum selon la relation suivante :

$$m_{OC} = \frac{m_{b,in}}{Y_{b,OC}}$$

dans lequel :

$m_{oc}$ indique la masse de carbone organique à ajouter au réacteur hétérotrophe au début de l'étape de culture au carbone organique,
$m_{b,n}$ indique la biomasse présente dans le réacteur hétérotrophe au début de l'étape de culture avec le carbone organique (masse d'inoculum),
$Y_{b,OC}$ indique la production en g de biomasse de microalgues produite pour un g de carbone organique utilisé, elle est spécifique à la souche de microalgues et au type de carbone organique ajouté.

**14.** Procédé selon l'une quelconque des revendications 10 à 12, dans lequel à la suite de l'addition du substrat de carbone organique, la culture est maintenue pendant une durée allant de 1 à 30 jours, sous agitation constante, à un pH entre 4 et 12 et une température entre 0 et 40°C, avec un approvisionnement en air continu, jusqu'à ce que le carbone organique en solution soit épuisé.

**15.** Procédé selon l'une quelconque des revendications 10 à 14, dans lequel à la fin de l'étape de culture avec du carbone organique, l'étape de culture à l'azote démarre en ajoutant un volume de solution en provenance du réservoir de stockage d'azote, dans lequel la quantité d'azote ajouté est entre 5 et 100 mg pour chaque gramme de biomasse présente dans le réacteur, où la circulation est maintenue pendant une durée entre 1 et 7 jours, sous agitation constante, à un pH entre 4 et 12 et la température entre 0 et 40°C, avec un approvisionnement en air continu, jusqu'à ce que l'azote en solution soit épuisé.

**16.** Procédé selon la revendication 7, dans lequel dans le mode d'alimentation discontinue le substrat d'azote et le substrat de carbone organique sont ajoutés directement sous forme solide dans le réacteur.

**17.** Procédé selon la revendication 1, dans lequel l'étape de culture hétérotrophe à alimentation discontinue de microalgues en c) est mise en œuvre comme décrit dans les revendications 7 à 16 en référence à la culture hétérotrophe lors de l'étape de démarrage a).

**18.** Procédé selon la revendication 17, dans lequel lors de l'étape de culture hétérotrophe en c) l'étape de culture au carbone organique et l'étape de culture à l'azote alternent jusqu'à atteindre la concentration et/ou la quantité de biomasse prédéterminées, et ladite étape de culture hétérotrophe se termine par une culture avec du carbone organique.

**19.** Procédé selon la revendication 1, dans lequel l'extraction de l'étape e) est mise en œuvre à partir d'une biomasse de microalgues obtenue lors de l'étape c) possédant un point d'humidité entre 70 et 90 % dans un réacteur par extraction en présence d'un solvant organique polaire complètement miscible dans l'eau, dans lequel la suspension est laissée sous agitation pendant 5 à 10 minutes à une température allant de 25 à 35°C, ledit solvant organique polaire complètement miscible dans l'eau étant un alcool ou un polyalcool à chaîne courte (C < 6), de préférence le méthanol, l'éthanol, le glycérol, le propanol, l'isopropanol ou des mélanges de ceux-ci, et dans lequel le solvant et la biomasse sont respectivement dans un rapport de 100 ± 20 l de solvant par kg de poids sec de biomasse.

**20.** Procédé selon la revendication 1, dans lequel l'extraction de l'étape g) est mise en œuvre dans un réacteur d'extraction en présence d'un mélange d'un solvant polaire et d'un solvant apolaire, dans lequel la suspension de biomasse est laissée pendant une heure à une température entre 25 et 35°C.

21. Procédé selon la revendication 20, dans lequel le mélange de solvant polaire et de solvant apolaire est sélectionné à partir du groupe constitué par :
l'hexane-2-propanol, l'hexane-méthanol, le dichlorométhane-méthanol, l'hexane-méthanol, le dichlorométhane-éthanol, et dans lequel le solvant et la biomasse sont respectivement dans un rapport de 500 ± 100 l de solvant par kg de poids sec de biomasse.

22. Procédé selon la revendication 1, dans lequel l'extraction d'amidon de l'étape i) a lieu dans un réacteur à micro-ondes irradié capable de générer une fréquence optimale de 2,45 GHz, et une puissance allant de 5 à 50 KW/l, de préférence égale à 33 KW/l, dans lequel de l'eau est ajoutée, au moins 10 l pour chaque kg de poids sec de biomasse, et est menée pendant une durée dépassant 5 minutes, de préférence pendant 60 minutes.

Figure 1

Figure 2

Figure 3

Figure 4

Figure 5

Figure 6

Figure 7

Figure 8

Figure 9

Figure 10

**EP 3 498 855 B1**

**REFERENCES CITED IN THE DESCRIPTION**

**Patent documents cited in the description**

- US 2012315678 A
- US 2009075353 A
- WO 2017130106 A